(19) Europäisches Patentamt

European Patent Office

Office européen des brevets

(11) **EP 0 716 864 B1**

(12) **EUROPÄISCHE PATENTSCHRIFT**

(45) Veröffentlichungstag und Bekanntmachung des Hinweises auf die Patenterteilung:
**10.04.2002 Patentblatt 2002/15**

(51) Int Cl.$^7$: **A61N 1/365**, A61N 1/368, A61B 5/0402, A61B 5/0472

(21) Anmeldenummer: **95118519.8**

(22) Anmeldetag: **24.11.1995**

(54) **Herzschrittmacher mit verbesserter Detektion elektrischer Signale**

Pacemaker with improved detection of electrical signals

Stimulateur cardiaque avec détection améliorée des signaux électriques

(84) Benannte Vertragsstaaten:
**DE ES FR GB IT**

(30) Priorität: **12.12.1994 DE 4444144**

(43) Veröffentlichungstag der Anmeldung:
**19.06.1996 Patentblatt 1996/25**

(73) Patentinhaber: **St. Jude Medical AB
175 84 Järfälla (SE)**

(72) Erfinder: **Hauptmann, Werner, Dipl.-Ing.
D-81673 München (DE)**

(56) Entgegenhaltungen:
**EP-A- 0 255 348      US-A- 4 616 659
US-A- 4 793 361      US-A- 4 799 486**

**Beschreibung**

Eine der kritischen und zugleich wichtigsten Komponenten eines Herzschrittmachers stellt das sogenannte Detektionssystem, im Englischen auch als "Sensing Circuit" bezeichnet, dar. Dieses System hat die Aufgabe, zwischen den abgegebenen Schrittmacherimpulsen die elektrische Aktivität des Herzens über die Schrittmachersonden laufend zu überwachen. In Abhängigkeit von der beobachteten Aktivität werden dann die Aktionen des Herzschrittmachers gesteuert, d. h. es werden Generatorimpulse ausgelöst oder unterbunden.

Obwohl es sich hier also um eine entscheidende Komponente in der Funktion des Gesamtsystems handelt, sind in derzeit verfügbaren Herzschrittmachern nur sehr einfache Verfahren und Einrichtungen zur Signalerkennung und Störunterdrückung realisiert. Zur Erkennung eines QRS-Komplexes werden oft nur einfache Schwellwertentscheider benutzt; bei manchen Geräten wird immerhin zusätzlich die Anstiegszeit des Signals ausgewertet. Zur Störunterdrückung werden zum Teil einfache Bandpaßfilter verwendet, um Störsignale zu unterdrücken, die eine hohe Amplitude aufweisen aber in einem anderen Frequenzbereich liegen. Der dem Fachmann bekannte Stand der Technik und das grundlegende Fachwissen auf diesem Gebiet sind z.B. in den Veröffentlichungen [1], [2], [3] und [4] wiedergegeben.

Diese Vorgehensweisen sind jedoch aus verschiedenen Gründen unzulänglich. Im Falle der Detektion von Ereignissen mit Hilfe von Signalamplituden oder Anstiegszeiten allein kann es einerseits dazu kommen, daß ein vorhandenes schwächeres QRS-Signal nicht erkannt wird, während andererseits ein starker Störimpuls als Herzsignal interpretiert wird. Weiterhin ist der Bereich des Frequenzbandes, in dem der QRS-Komplex liegt unterschiedlich bei verschiedenen Patienten, er verschiebt sich sogar für verschiedene Herzschläge eines einzelnen Patienten. Aufgrund der verwendeten sehr einfachen Methoden kommt es bei derzeit realisierten Systemen gelegentlich zu Fehlverhalten und Funktionsstörungen aufgrund von Störsignalen, Übersprechen oder zu schwacher elektrischer Herzaktivität.

Ziel der Erfindung ist es, die Leistungsfähigkeit und Robustheit des Detektionssystems eines Herzschrittmachers zu steigern. Dies geschieht durch die Erweiterung des Herzschrittmachers um ein Verarbeitungssystem zur verbesserten Detektion definierten Signalereignissen, d.h. auch zur verbesserten Unterdrückung von internen und externen Störungs- und Rauscheinflüssen. Erfindungsgemäß ist daher ein Herzschrittmacher mit verbesserter Detektion elektrischer Signale vorgesehen, mit Mitteln zur Aufnahme intrakardialer Herzsignale, in welchen den zu detektierenden Signalen gewissen Rauschsignale mit einem im allgemeinen farbigen Spektrum überlagert sind, mit Mitteln zur adaptiven, nichtlinearen Rauschfilterung dieser intrakardialen Herzsignale, welche das Spektrum der Rauschsignale in ein im wesentlichen weißes Spektrum überführen, sowie mit Mitteln zur angepaßten Filterung (Matched Filtering, MF) der durch die nichtlineare Rauschfilterung gefilterten Signale durch Korrelation dieser Signale mit einem Signalmuster. Schließlich wird eine Ausgabegröße erzeugt, deren Betrag ein Maß für das Vorhandensein dieses Signalmusters in dem Herzsignal ist. Im Gegensatz zu bekannten Herzschrittmachern verwendet die Erfindung eine adaptive nichtlineare Rauschfilterung zur Überführung des Spektrums der Störsignale in ein im wesentlichen weißes Spektrum. Hierdurch wird die Detektion bestimmter Signalmuster mit Hilfe angepaßter Filter MF entscheidend verbessert, da diese angepaßten Filter nur unter der Voraussetzung optimal arbeiten, daß das Rauschspektrum im wesentlichen weiß ist.

Bei einer bevorzugten Ausführungsform der Erfindung wird die erzeugte Ausgabegröße mit Hilfe einer Schwellwertentscheidung in eine binäre Größe umgewandelt. Hierdurch kann die binäre Größe direkt zur Steuerung weiterer Herzschrittmacherfunktionen, z.B. mit Hilfe hierfür geeigneter digitaler Schaltungen, verwendet werden. Diese binäre Größe kann gemäß einer weiteren bevorzugten Ausführungsform der Erfindung zur Erzeugung eines Triggerimpulses verwendet werden, welcher zur Steuerung von Herzschrittmacherfunktionen verwendet wird, und ausgegeben wird, sobald die Ausgabegröße eine vorgegebene Schwelle übersteigt.

Bei einer weiteren bevorzugten Ausführungsform der Erfindung wird das Triggersignal zur Extraktion einer Stichprobe eines gesuchten Signalmusters aus dem intrakardialen Herzsignal verwendet. Diese Stichprobe wird anschließend zur zeitlichen Adaption des Signalmusters verwendet.

Die Erfindung eignet sich insbesondere zur Detektion des QRS-Komplexes im intrakardialen Herzsignal.

Im folgenden wird die Erfindung mit Hilfe bevorzugter Ausführungsbeispiele und anhand der Figuren näher beschrieben.

Figur 1   zeigt ein Blockschaltbild der erfindungsgemäßen adaptiven Detektion von bestimmten Signalereignissen mit Hilfe angepaßter Filter MF am Beispiel des QRS-Komplexes.

Figur 2   zeigt ein Blockschaltbild eines adaptiven neuronalen Rauschfilters NN/FIR, wie es im Zusammenhang mit der Erfindung bevorzugt verwendet wird.

Figur 3   zeigt in schematischer Weise die Struktur eines herkömmlichen linearen adaptiven FIR-Filters.

Figur 4   zeigt in schematischer Weise ein nichtlineares, adaptives, neuronales Filter NN/FIR, wie es im Zusammenhang mit der Erfindung bevorzugt verwendet wird.

Es ist das Ziel der Erfindung die Leistungsfähigkeit und Robustheit des Detektionssystems eines Herzschrittmachers zu steigern, indem das Herzschrittmachersystem um ein Verarbeitungssystem zur verbesserten Detektion von definierten Signalereignissen, d.h. auch zur verbesserten Unterdrückung von internen und externen Störungs- und Rauscheinflüssen erweitert wird.

Eine der Hauptfunktionen des Detektionssystems eines Herzschrittmachers ist die ständige zuverlässige Erkennung des vom Herzen erzeugten QRS-Komplexes. Je nach dem ob ein solches QRS-Signal empfangen wurde oder nicht, erfolgt kurz darauf vom Generator des Herzschrittmachers eine Impulsauslösung zur Reizung des Herzens. Die sichere und fehlerfreie Funktion des Detektors hängt also unmittelbar davon ab, ob

- ein vorgegebenes, wohl definiertes Signalereignis jedes Mal sicher erkannt wird, wenn es auftritt, und ob

- alle anderen Signalereignisse, auch wenn sie eine hohe Energie oder Ähnlichkeit zum gewünschten Signalereignis aufweisen, als Störsignal erkannt und damit unterdrückt werden.

Als definierte Signalereignisse kommen in diesem Zusammenhang z. B. der QRS-Komplex oder auch ein definiertes pathologisches Herzschlag-Muster in Frage. Typische Störsignale im Sinne dieser Beschreibung sind z. B. externe Störungen oder internes Übersprechen bei Zweikammersystemen. Eine sichere Entscheidung des Detektors ist von erheblicher Bedeutung für die zuverlässige Funktion des Herzschrittmachers. Wird z. B. ein QRS-Signal nicht erkannt, obwohl es vorhanden war, feuert der Herzschrittmacher in den bereits natürlich ablaufenden Reizleitungsvorgang hinein. Wird dagegen ein QRS-Signal festgestellt, obwohl es sich dabei um eine externe Störung handelt und kein natürlicher Reiz des Herzens vorhanden war, wird die Abgabe des Schrittmacherimpulses unterbunden - es erfolgt kein Herzschlag. In beiden Fällen kann es zu lebensgefährlichen Herzrythmusstörungen kommen.

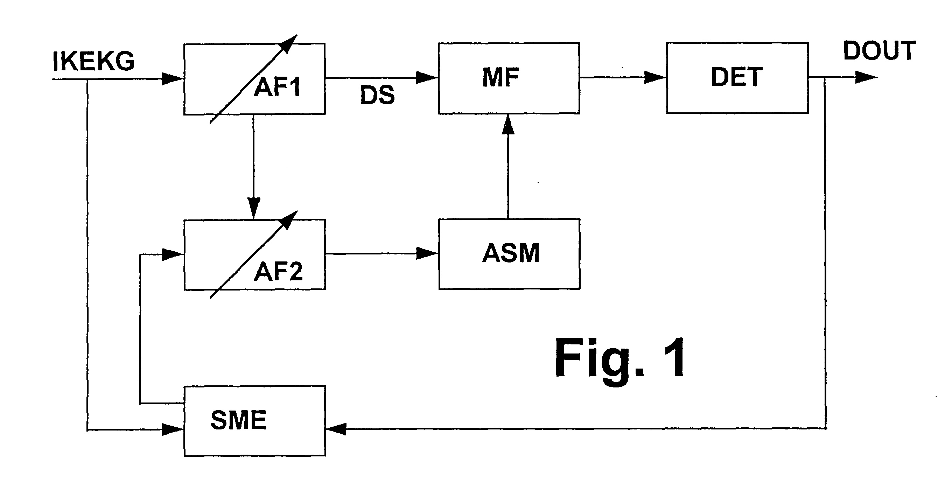

Da die fehlerfreie Detektion des QRS-Komplexes die wichtigste Aufgabe des Detektionssystems des Herzschrittmachers ist, wird im folgenden die Funktion der Signaldetektion für den Fall der QRS-Erkennung dargestellt. Der Fachmann ist jedoch anhand der hier gegebenen Beschreibung der Erfindung ohne weiteres in der Lage, die Erfindung auch zur Detektion anderer Signalereignisse, z. B. definierter pathologischer Herzschlagmuster zu verwenden. Das hier beschriebene, für die Verarbeitung von intrakardialen Herzsignalen vorgesehene, Detektionsverfahren beruht auf der Verknüpfung von Ansätzen aus der klassischen Signalverarbeitung (Matched-Filter, LMS-Algorithmus) mit Ansätzen aus dem Gebiet der neuronalen Netze (nicht lineares adaptives Rauschfilter). Figur 1 zeigt schematisch den Aufbau eines erfindungsgemäßen Verarbeitungssystems zur adaptiven Signaldetektion.

Das Eingangssignal des Detektorsystems ist das sogenannte intrakardiale EKG, das laufend über die Herzschrittmachersonde beobachtet wird. Bei dem Problem der Signaldetektion hat man ganz allgemein das Ziel, das Signalereignis s(t) im empfangenen Signal x(t), dem sich das Rauschen n(t) additiv überlagert,

$$(1) \qquad x(t) = s(t) + n(t)$$

zu erkennen. Für den Fall der Herzschlagerkennung im EKG stellt das Signal s(t) den QRS-Komplex dar und das Rauschen n(t) repräsentiert alle anderen Komponenten des EKGs einschließlich der P- und T-Wellen, des Rauschens der Schaltkreise und Instrumente sowie der Störungen die durch Muskelaktivität und andere Vorgänge verursacht werden. Was das EKG-Signal betrifft, verfügt man über Vorwissen über das relevante Signalereignis in Form eines typischen QRS-Signalmusters (Template). Da die tatsächliche Form des QRS-Signals jedoch einerseits zwischen verschiedenen Patienten differieren und andererseits auch für ein und denselben Patienten zeitlichen Schwankungen unterliegen kann, wird außerdem eine automatische Anpassung des momentan gültigen Signalmusters vorgenommen. Diese Anpassung wird im weiteren Verlauf dieser Patentanmeldung noch beschrieben werden. Das Vorwissen über das Signalereignis nutzt der erfindungsgemäße Signaldetektor aus durch die Verwendung eines angepaßten Filters (Matched Filter MF), dessen Impulsantwort an das gesuchte Signalereignis (Signalmuster) angepaßt ist. Es erfolgt eine fortlaufende Korrelation zwischen dem hereinkommenden Signal und dem Testmuster. Das Filter kann durch ein digitales FIR-Filter (Finite Impulse Response, Filter mit endlicher Impulsantwort) realisiert werden, welches die Impulsantwort $h(t) = s(t-t_0)$ besitzt, so daß das Signal-zu-Rauschverhältnis am Ausgang des Filters maximal ist, wenn x(t) auf den Eingang gegeben wird. Dabei ist das Signal-Rauschverhältnis (SNR) definiert durch

$$(2) \qquad SNR = \frac{|y_s(t_0)|}{\sqrt{E\{y_n^2\}}}$$

In dieser Gleichung ergibt sich die Antwort des Filters auf den Signalanteil zu

$$(3) \qquad y_s(t_0) = s(t) * h(t)$$

als Resultat der Faltung des Signals (z. B. des QRS-Komplex) mit der Impulsantwort des Filters. In ähnlicher Weise ergibt sich die Filterantwort auf den Rauschanteil zu

$$(4) \qquad y_n(t_o) = n(t) * h(t)$$

Das beschriebene angepaßte Filter MF (Matched Filter) verhält sich nur dann als optimaler Detektor, wenn es sich bei dem Stör- bzw. Rauschsignal n(t) um ein stationäres weißes Rauschen handelt. Dies ist jedoch bei der Detektion des QRS-Komplexes nicht gegeben. Dem interessierenden Nutzsignal ist hier vielmehr ein farbiges Rauschsignal überlagert, welches Teile des EKG-Signals, z. B. die sogenannte P- und T-Welle, das Rauschen der Instrumente und durch Muskelaktivitäten hervorgerufene Störungen beinhaltet. Bei diesen Komponenten handelt es sich oft um korrelierte, stark nichtlineare und nichtstationäre Rauschprozesse, sogenanntes farbiges Rauschen. Um das erfindungsgemäße Detektionsverfahren mit dem angepaßten Filter in diesem Fall anwenden zu können, muß vor der Korrelation mit dem Testmuster eine Vorverarbeitung durch ein Rauschfilter bzw. ein Dekorrelationsfilter (Whitening Filter) erfolgen, mit dem die korrelierten Anteile des Rauschens unterdrückt werden.

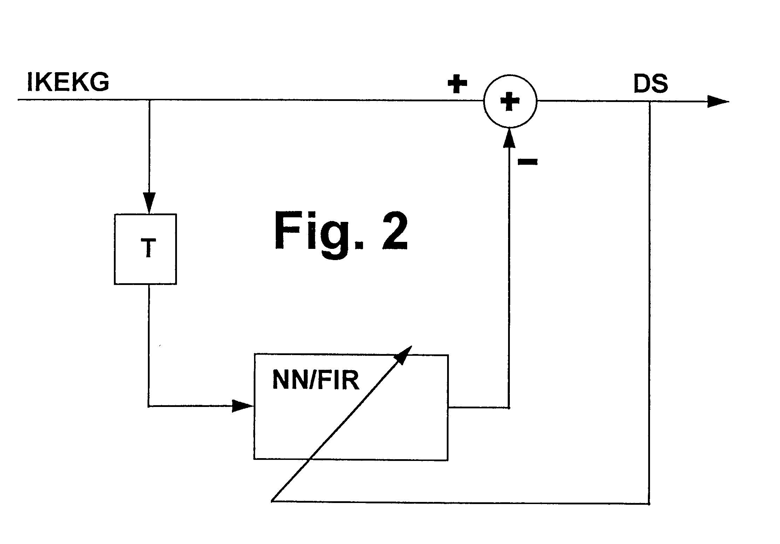

Für die Modelierung des überlagerten korrelierten Rauschprozesses wird üblicherweise ein autoregressives Modell (AR-Modell) zugrunde gelegt. Entsprechend kann damit das Rauschfilter als lineares adaptives Filter aufgebaut werden, dessen Koeffizienten sich gemäß den jeweils berechneten AR-Parametern einstellen. Bei dem hier vorliegenden Problem sind die Hintergrundrauschprozesse jedoch stark nichtlinear und deshalb ist der Einsatz eines solchen linearen Filters nicht sehr wirksam. Zur verbesserten Rauschprozeßmodellierung sieht die Erfindung deshalb eine Methode der Signalfilterung vor, die auf dem Prinzip eines neuronalen Netzes basiert. Dazu wird das übliche lineare adaptive Dekorrelationsfilter durch ein adaptives nichtlineares Rauschfilter ersetzt. Figur 2 zeigt in schematischer Weise das Blockschaltbild eines solchen neuronalen Filters NN, das bis auf die eigentliche Verarbeitungsstufe NN/FIR, dem eines herkömmlichen linearen adaptiven Filters entspricht.

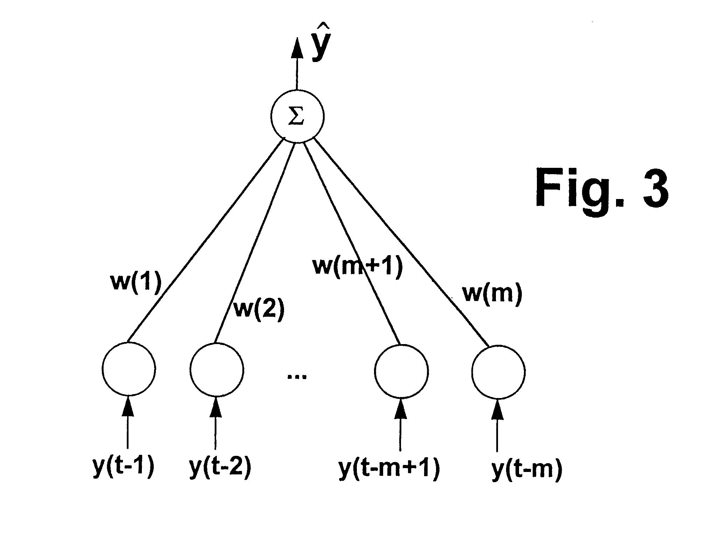

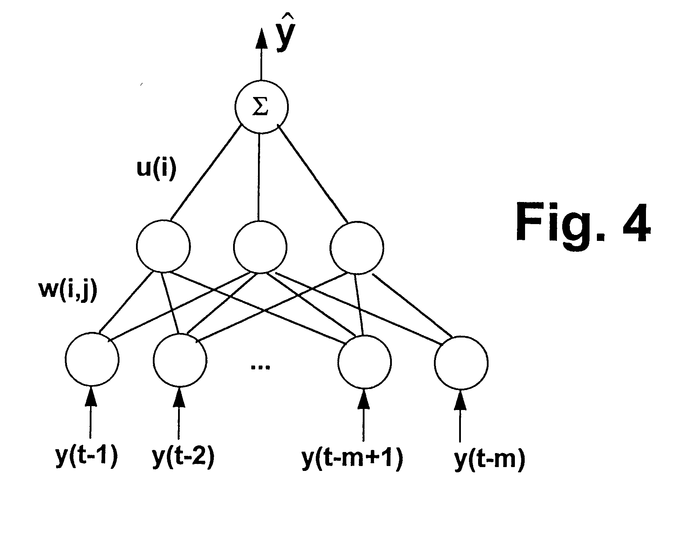

In Figur 4 ist die Struktur dieser Verarbeitungsstufe NN/FIR des neuronalen FIR-Filters nochmals im Detail schematisch dargestellt und kann so der entsprechenden Struktur eines herkömmlichen linearen adaptiven Filters, wie es in Figur 3 dargestellt ist, gegenübergestellt werden. Die Grundstruktur des neuronalen adaptiven Filters NN besteht aus drei Schichten: einer Eingabeschicht, einer Ausgabeschicht und einer verborgenen Schicht (Hidden-Layer). Das nichtlineare adaptive neuronale Filter kann man sich auch als eine Erweiterung des linearen Filters um eine verborgene Schicht vorstellen. Diese Schicht besteht aus nichtlinearen Verarbeitungsknoten, die an ihren Ausgängen die Zwischenergebnisse

$$(5) \qquad Z_i = f\left(\sum_{j=1}^{M} w_{ij} y_{t-i} + b_j\right)$$

liefern. Als Aktivierungsfunktion f( ) wird eine Sigmoidfunktion benutzt. Mit $w_{ij}$ sind die Gewichte bezeichnet, die die Eingangsknoten mit den Knoten der verborgenen Schicht verbinden; die Größen $b_{ij}$ sind die sogenannten Offset-Werte. Der Ausgang der Verarbeitungsstufe NN/FIR des neuronalen Filters ergibt sich als Linearkombination der Ausgangswerte der verborgenen Schicht. Als Ausgang des gesamten Dekorrelationsfilters, wie es in Figur 2 dargestellt ist, ergibt sich somit die Größe $y_0$ zu

$$(6) \qquad y_0 = y_t - \hat{y} = y_t - \sum_{i=1}^{q} u_i z_{t-i} = y_t - \sum_{i=1}^{q} u_i f\left( \sum_{j=1}^{M} w_{ij} y_{t-i} + b_j \right)$$

Mit $u_i$ sind hier die Gewichte zwischen der verborgenen Schicht und dem Ausgangsknoten bezeichnet. Dieser Modellierungsfilter bildet den Ausgangspunkt für das Trainieren des neuronalen Netzes, welches nach der Methode der sogenannten "Backpropagation" ununterbrochen durchgeführt wird. Das Netz hat nur einen Ausgang, welcher den modellierten Rauschprozeß liefert, die Anzahl der Knoten der verborgenen Schicht ist variabel. Die Zahl der Eingänge des neuronalen Netzes entspricht der Ordnung des Filters und muß entsprechend gewählt werden. Dem Fachmann ist es mit Hilfe seines Fachwissens möglich, die Filterordnung und damit die Zahl der Eingänge jeweils dem Problem entsprechend angemessen zu wählen.

Die fortlaufende Adaption an den aktuellen Rauschprozeß und die Nichtlinearität des neuronalen Signalfilters gestatten eine genauere Modellierung der zugrundeliegenden Signalcharakteristik. Damit wird der am Rauschfiltereingang anliegende korrelierte Rauschanteil des Signals x(t) dekorreliert, d. h. er weist nach der Filterung eine weiße Spektralcharakteristik auf, und kann dem Eingang des nachfolgenden angepaßten Filters zugeführt werden.

Im folgenden werden noch einmal die wesentlichen Verarbeitungsschritte des adaptiven Detektionsalgorithmus anhand des Blockschaltbildes (Figur 1) zusammenfassend dargestellt:

Über den Detektoreingang wird das elektrische Signal, das über die Herzschrittmachersonde aufgenommen wird, das intrakardiale EKG, dem adaptiven nichtlinearen neuronalen Rauschfilter zugeführt. Dieses Rauschfilter modelliert den momentanen Rauschprozeß des Signals und adaptiert entsprechend seine Gewichte und Filterkoeffizienten. Nach kurzer Adaptionszeit liegt am Ausgang des Rauschfilters ein Signal mit überlagertem Rauschprozeß vor, der nahezu weiße Spektralcharakteristik aufweist.

Dieses Signal wird auf das angepaßte Filter gegeben, dessen Filterkoeffizienten entsprechend dem zu detektierenden Signalereignis (z. B. dem QRS-Komplex) voreingestellt sind. Hier erfolgt eine Korrelation des hereinkommenden Signals mit dem gesuchten Signalmuster. Am Ausgang des angepaßten Filters stellt sich nur dann ein hoher Wert ein, wenn ein entsprechender QRS-Komplex detektiert wurde. Im anschließenden QRS-Detektor wird eine einfache Schwellwertentscheidung getroffen und ein kurzer Triggerimpuls auf den Detektorausgang gegeben, sobald eine vorgegebene Schwelle überschritten wird. Dieser Triggerimpuls kann dazu benutzt werden, einen Herzschrittmacherpuls zu erzeugen oder zu unterbinden.

Bei einer bevorzugten Ausführungsform der Erfindung wird zusätzlich zum Rauschfilter auch noch das gesuchte Signalmuster laufend mit der Zeit adaptiert. Hierdurch ist eine Anpassung an zeitlich veränderliche Signalmuster (z. B. zeitlich veränderte QRS-Komplexe) möglich. Da der genaue Verlauf eines QRS-Signals zeitlichen Veränderungen unterliegt, wird hierdurch eine gleichbleibend hohe Erkennungsrate des Detektors ermöglicht. Außerdem wird damit eine individuelle, auf den Patienten abgestimmte Einstellung ermöglicht. Dazu wird das Eingangssignal der Verarbeitungsstufe Signalmusterextraktion zugeführt. Hier wird aus dem laufend eingespeisten Originalsignal in einstellbaren Intervallen mit Hilfe des Triggersignal des Detektorausgangs der aktuelle Verlauf des QRS-Signals extrahiert und in einem weiteren adaptiven Rauschfilter, das identisch zum Filter am Detektoreingang ist, dekorreliert. Im Verarbeitungsblock "Aktuelles Signalmuster" sind N Signalmuster aus den vorhergehenden Aktualisierungen gespeichert und das aktuelle Signalmuster, das für die Matched-Filterung benutzt wird, berechnet sich aus der Mittelung über diese N jüngsten Muster nach der Formel

$$(7) \qquad QRS = \frac{1}{N} \sum_{i=1}^{N} QRS_i(t)$$

Jeweils bei der Aktualisierung wird das älteste Signalmuster gelöscht und das neue aktuelle QRS-Muster nach dieser Beziehung berechnet. Je nach Wahl der Anzahl von vergangenen Signalmustern N kann man festlegen, wie stark der Einfluß des neuesten Signalereignisses sein soll.

Dem Fachmann ist anhand der gegebenen Beschreibung der Erfindung klar, daß die Erfindung nicht ausschließlich auf die Detektion des QRS-Komplexes beschränkt ist. Mit Hilfe der Erfindung können auch andere charakteristische Signalereignisse detektiert werden, und so der Patient z. B. bei der Erkennung von gefährlichen Signalereignissen gewarnt werden.

Im Rahmen dieser Patentanmeldung wurden die folgenden Veröffentlichungen zitiert:

# EP 0 716 864 B1

[1] Moses, H.W. et al., "A Practical Guide to Cardiac Pacing", Little, Brown and Company, Boston, 1991, 3rd ed.

[2] Gülker H. et al., "Leitfaden zur Therapie der Herzrhythmusstörungen", pp. 196 - 231, de Gruyter, 1992, ". Aufl.

[3] Haykin, S., "Adaptive Filter Theory", Prentice Hall Intl, Inc., Englewood Cliffs, 1991

[4] van Trees, H.L., "Detection, Estimation, and Linear Modulation Theory", Wiley & Sons, New York, 1968

**Patentansprüche**

1. Herzschrittmacher mit verbesserter Detektion elektrischer Signale mit folgenden Merkmalen:

   es sind Mittel vorgesehen zur

   a) Aufnahme intrakardialer Herzsignale, in welchen den zu detektierenden Signalen gewisse Rauschsignale mit einem im allgemeinen farbigem Spektrum überlagert sind; **gekennzeichnet, dadurch daß** Mittel vorgesehen sind zur
   b) nichtlinearen adaptiven neuronalen Rauschfilterung dieser intrakardialen Herzsignale, welche das Spektrum der Rauschsignale in ein im wesentlichen weißes Spektrum überführen;
   c) angepaßten Filterung, d.h. zum Matched Filtering der durch die nichtlineare Rauschfilterung gefilterten Signale durch Korrelation dieser Signale mit einem gesuchten Signalmuster und Erzeugung einer Ausgabegröße, deren Betrag ein Maß für das Vorhandensein dieses gesuchten Signalmusters in dem Herzsignal ist.

2. Herzschrittmacher nach Anspruch 1, bei dem eine Binarisierung dieser Ausgabegröße mit Hilfe einer Schwellwertentscheidung erfolgt.

3. Herzschrittmacher nach Anspruch 2, bei dem ein Triggerimpuls zur Steuerung von Herzschrittmacherfunktionen ausgegeben wird, sobald die Ausgabegröße eine vorgegebene Schwelle übersteigt.

4. Herzschrittmacher nach Anspruch 3, bei dem das Triggersignal auch zur Extraktion einer Stichprobe eines gesuchten Signalmusters aus dem intrakardialen Herzsignal verwendet wird, und bei dem diese Stichprobe zur zeitlichen Adaption dieses Signalmusters verwendet wird.

5. Herzschrittmacher nach einem der vorhergehenden Ansprüche, bei dem der QRS-Komplex ein gesuchtes Signalmuster ist.

**Claims**

1. Cardiac pacemaker with improved detection of electrical signals with the following features:

   means are provided for

   a) receiving intracardial cardiac signals, in which certain noise signals with a generally coloured spectrum are superimposed on the signals to be detected; **characterised in that** means are provided for
   b) non-linear adaptive neuronal noise filtering of these intracardial cardiac signals, which converts the spectrum of the noise signals into a substantially white spectrum;
   c) matched filtering of the signals filtered by the non-linear noise filtering by correlation of the signals with a desired signal pattern and production of an output parameter, the value of which is a measure of the presence of this desired signal pattern in the cardiac signal.

2. Cardiac pacemaker as claimed in Claim 1, in which this output parameter is digitised with the aid of a threshold decision.

3. Cardiac pacemaker as claimed in Claim 2 in which a trigger pulse for controlling the cardiac pacemaker functions is produced as soon as the output parameter exceeds a predetermined threshold value.

4. Cardiac pacemaker as claimed in Claim 3, in which the trigger signal is also used to extract a sample of a desired signal pattern from the intracardial cardiac signal and in which this sample is used for temporal adaption of this signal pattern.

5. Cardiac pacemaker as claimed in one of the preceding claims, in which the QRS complex is a desired signal pattern.

**Revendications**

1. Stimulateur cardiaque avec détection améliorée de signaux électriques, comportant les caractéristiques suivantes :

   des moyens sont prévus

   a) pour la réception de signaux intracardiaques dans lesquels certains signaux de bruit ayant un spectre généralement coloré sont superposés aux signaux à détecter ; **caractérisé par le fait que** des moyens sont prévus
   b) pour le filtrage de bruit adaptatif neuronal non linéaire de ces signaux intracardiaques en transférant le spectre des signaux de bruit dans un spectre sensiblement blanc ;
   c) pour le filtrage adapté, ou Matched Filtering, des signaux, filtrés par le filtrage de bruit non linéaire, par corrélation de ces signaux avec un modèle de signal recherché et pour la production d'une grandeur de sortie dont la valeur est une mesure pour la présence de ce modèle de signal recherché dans le signal cardiaque.

2. Stimulateur cardiaque selon la revendication 1, dans lequel cette grandeur de sortie est transformée en une valeur binaire à l'aide d'un dispositif de décision à valeur de seuil.

3. Stimulateur cardiaque selon la revendication 2, dans lequel une impulsion de déclenchement est délivrée pour la commande de fonctions du stimulateur cardiaque dès que la grandeur de sortie dépasse un seuil prédéterminé.

4. Stimulateur cardiaque selon la revendication 3, dans lequel le signal de déclenchement est aussi utilisé pour l'extraction d'un échantillon d'un modèle de signal recherché hors du signal intracardiaque et dans lequel cet échantillon est utilisé pour l'adaptation temporelle de ce modèle de signal.

5. Stimulateur cardiaque selon l'une des revendications précédentes, dans lequel le complexe QRS est un modèle de signal recherché.

Fig. 1

Fig. 2

EP 0 716 864 B1

# Fig. 3

$\hat{y}$

$\Sigma$

$w(1)$ — $y(t-1)$

$w(2)$ — $y(t-2)$

$w(m+1)$ — $y(t-m+1)$

$w(m)$ — $y(t-m)$

...

Fig. 4